# EUROPEAN PATENT APPLICATION

(11) **EP 1 466 612 A1**
(43) Date of publication of application: **13.10.2004**
(21) Application number: 03076023.5
(22) Date of filing: 08.04.2003
(51) Int. Cl.: A61K 38/06, A61K 38/07, A61K 38/24, A61P 29/00

(54) **Treatment of inflammation and sepsis with hCG derived peptides**

(71) Applicant: Biotempt B.V., 7958 NZ Koekange (NL)
(72) Inventor: The designation of the inventor has not yet been filed

(57) **Abstract**

The invention provides use of a gene-regulatory peptide or functional analogue thereof for the production of a pharmaceutical composition for the treatment of a disease comprising an inflammatory condition and/or a counter-inflammatory condition wherein a subject suffering from said disease is subjected to a diagnostic process aimed at determining inflammatory disease stage of said subject and where treatment is selected depending on the outcome of said determination of disease stage.

## Description

### Technical field

The current invention relates to the body's innate way of modulation of important physiological processes and builds on insights reported in WO99/59617, WO01/72831 and PCT/NL02/00639.

### Background

In these earlier applications small gene-regulatory peptides are described that are present naturally in pregnant women and are derived from proteolytic breakdown of placental gonadotropins such as human chorionic gonadotropin (hCG) produced during pregnancy. These peptides (in their active state often only at about 4 to 6 amino acids long) were shown to have unsurpassed immunological activity that they exert by regulating expression of genes encoding inflammatory mediators such as cytokines. Surprisingly, it was found that breakdown of hCG provides a cascade of peptides that help maintain a pregnant woman's immunological homeostasis. These peptides are nature's own substances that balance the immune system to assure that the mother stays immunologically sound while her foetus does not get prematurely rejected during pregnancy but instead is safely carried through its time of birth.

Where it was generally thought that the smallest breakdown products of proteins have no specific biological function on their own (except to serve as antigen for the immune system), it now emerges that the body in fact routinely utilises the normal process of proteolytic breakdown of the proteins it produces to generate important gene-regulatory compounds, short peptides that control the expression of the body's own genes. Apparently the body uses a gene-control system ruled by small broken down products of the exact proteins that are encoded by its own genes.

It is long known that during pregnancy the maternal system introduces a status of temporary immuno-modulation which results in suppression of maternal rejection responses directed against the foetus. Paradoxically, during pregnancy, often the mother's resistance to infection is increased and she is found to be better protected against the clinical symptoms of various auto-immune diseases such as rheumatism and multiple sclerosis. The protection of the foetus can thus not be interpreted only as a result of immune suppression. Each of the above three applications have provided insights by which the immunological balance between protection of the mother and protection of the foetus can be understood.

It was shown that certain short breakdown products of hCG (i.e. short peptides which can easily be synthesised, if needed modified, and used as pharmaceutical composition) exert a major regulatory activity on pro- or anti-inflammatory cytokine cascades that are governed by a family of crucial transcription factors, the NFkappaB family which stands central in regulating the expression of genes that shape the body's immune response.

Most of the hCG produced during pregnancy is produced by cells of the placenta, the exact organ where cells and tissues of mother and child most intensely meet and where immuno-modulation is most needed to fight off rejection. Being produced locally, the gene-regulatory peptides which are broken down from hCG in the placenta immediately balance the pro- or anti-inflammatory cytokine cascades found in the no-mans land between mother and child. Being produced by the typical placental cell, the trophoblast, the peptides traverse extracellular space; enter cells of the immune system and exert their immuno-modulatory activity by modulating NFkappaB-mediated expression of cytokine genes, thereby keeping the immunological responses in the placenta at bay.

### Summary of the invention

It is herein postulated that the beneficial effects seen on the occurrence and severity of auto-immune disease in the pregnant woman result from an overspill of the hCG-derived peptides into the body as a whole; however, these effects must not be overestimated, as it is easily understood that the further away from the placenta, the less immuno-modulatory activity aimed at preventing rejection of the foetus will be seen, if only because of a dilution of the placenta-produced peptides throughout the body as a whole. However, the immuno-modulatory and gene-regulatory activity of the peptides should by no means only be thought to occur during pregnancy and in the placenta; man and women alike produce hCG, for example in their pituitaries, and nature certainly utilises the gene-regulatory activities of peptides in a larger whole.

Consequently, a novel therapeutic inroad is provided, using the pharmaceutical potential of gene-regulatory peptides and derivatives thereof. Indeed, evidence of specific up- or down-regulation of NFkappaB driven pro- or anti-inflammatory cytokine cascades that are each, and in concert, directing the body's immune response was found *in silico* in gene-arrays by expression profiling studies, *in vitro* after treatment of immune cells and *in vivo* in experimental animals treated with gene-regulatory peptides. Also, considering that NFkappaB is a primary effector of disease (A.S. Baldwin, J. Clin. Invest., 2001, 107:3-6), using the hCG derived gene-regulatory peptides offer significant potential for the treatment of a variety of human and animal diseases, thereby tapping the pharmaceutical potential of the exact substances that help balance the mother's immune system such that her pregnancy is safely maintained.

### Detailed description of the invention

The invention in particular relates to methods of treatment of disease comprising an inflammatory condition and/or a counter-inflammatory condition.

Inflammation is a general name for reactions occurring after most kinds of tissue injuries or infections or immunologic stimulation as a defense against foreign or altered endogenous substances. Inflammatory reactions involve a number of biochemical and cellular alterations the extent of which correlates with the extent of the initial trauma (Wound healing). Inappropriate activation of inflammatory responses is the underlying cause of many common diseases and inflammatory reaction are, therefore, also an important target for drug development.

The most prominent systemic manifestation of inflammation is an elevation of body temperature and a variety of biochemical alterations known as the acute phase reaction which leads to the synthesis of acute phase proteins in the liver.

The local inflammatory reaction is characterized by an initial increase in blood flow to the site of injury, enhanced vascular permeability, and the ordered and directional influx and selective accumulation of different effector cells from the peripheral blood at the site of injury. Influx of antigen non-specific but highly destructive cells (neutrophils) is one of the earliest stages of the inflammatory response. These cells mount a rapid, non-specific phagocytic response.

At a later stage monocyte-macrophages and cells of other lymphocyte lineages (specific subsets of T-cells and B-cells) appear at the site of injury. These cell types are associated with antigen-specific and more tightly regulated immune responses and once activated also produce protective and inflammatory molecules. An exudation of plasma into the lesion in the early stage is observed also.

Inflammatory cells express increasing numbers of cell-surface proteins and glycoproteins known as cell adhesion molecules. Endothelial cells are also activated during the initial phase of the inflammatory response and then express, among other things, adhesion molecule counterreceptors. The regulated expression of these molecules allows for the precise trafficking of circulating leukocytes to inflammatory sites. Cellular attachment of immune cells to endothelial cells lining blood vessels surrounding the inflammatory site prevents them from being swept past the site of infection or tissue damage and is a crucial step required for the subsequent emigration of these cells into the surrounding inflammatory tissues (extravasation).

The highly efficient process of cellular influx to inflammatory sites is mediated by a plethora of mediator substances supporting and dispersing inflammation. These mediators are found in the serum or tissue fluids, are released by degranulating cells, and are secreted also by inflammatory cells upon activation, or activated endothelial cells in blood vessels at the site of inflammation. They serve as muscle-active and edema-promoting substances, chemotaxins, and cellular activators and inducers of all kinds of effector cells engaged in the inflammatory response.

Inflammatory mediators include some well studied compounds such as anaphylatoxins of the complement cascade, kinins of the coagulation system, leukotriens, prostaglandins, and many other lipid mediators. Another group of mediators are neuropeptides such as Tachykinins, VIP (vasoactive intestinal peptide), and VPF (vascular permeability factor). These substances enhance capillary permeability and have vasodilatatory and bronchoconstrictory activity and also increase the production of mucus.

A number of cytokines, known collectively aspro-inflammatory cytokines because they accelerate inflammation, also regulate inflammatory reactions either directly or by their ability to induce the synthesis of cellular adhesion molecules or other cytokines in certain cell types. The major pro-inflammatory cytokines that are responsible for early responses are IL1-alpha, IL1-beta, IL6, and TNF-alpha. Other pro-inflammatory mediators include LIF, IFN-gamma, OSM, CNTF, TGF-beta, GM-CSF, IL11, IL12, IL17, IL18, IL8 and a variety of other chemokines that chemoattract inflammatory cells, and various neuromodulatory factors. The net effect of an inflammatory response is determined by the balance between pro-inflammatory cytokines and anti-inflammatory cytokines (for example IL4, IL10, and IL13, IL16, IFN-alpha, TGF-beta, IL1ra, G-CSF, soluble receptors for TNF or IL6).

The observed redundancy among the different cytokines and other mediators of inflammation generally guarantees a substitution or complementation of individual components that may have been inactivated under pathological conditions.

Under normal circumstances these cascades of inflammatory reactions induced by the mediators are strictly regulated. Failure to do so can lead to multiple organ failure (Systemic inflammatory response syndrome). Inflammatory mediators and suitable inhibitors are, therefore, of key interest for modulating and ameliorating the effects of inflammatory reactions and their sequelae.

Sepsis/SIRS is an example of an acute systemic inflammatory response to a variety of noxious insults (particularly insults of an infectious origin such as a bacterial infection, but also non-infectious insults are well known and often seen). The systemic inflammatory response seen with sepsis /SIRS is caused by immunological processes that are activated by a variety of immunological mediators such as cytokines, chemokines, nitric oxide, and other immune mediating chemicals of the body. These immunological mediators are generally seen to cause the life-threatening systemic disease seen with sepsis/SIRS. These immunological mediators are, one the one hand, required locally, for example as effective antibacterial response, but are, in contrast, potentially toxic when secreted into the circulation. When secreted into the circulation, these mediators can cause, in an upward spiral of cause and effect, the further systemic release of these mediators, in the end leading to severe disease, such as multiple organ failure and death.

Central in the development of sepsis/SIRS in a subject is the presence and effects of immunological mediators that give rise to a disease that pertains to or affects the body as a whole, a systemic disease. This systemic immunological response can be caused by a variety of clinical insults, such as trauma, burns and pancreatitis. The phrase "systemic" inflammatory response syndrome (SIRS)" has been introduced to designate the signs and symptoms of patients suffering from such a condition. SIRS has a continuum of severity ranging from the presence of tachycardia, tachypnea, fever and leukocytosis, to refractory hypotension and, in its most severe from, shock and multiple organ system dysfunction.

The crucial pathophysiologic event that precipitates systemic inflammation is tissue damage. This can occur both as a result of the direct injury to tissues from mechanical or thermal trauma as well as cellular injury induced by mediators of ischemia-reperfusion injury such as oxygen free radicals. Injury results in the acute release of proinflammatory cytokines. If injury is severe, such as in extensive thermal injury, a profound relase of cytokines occurs, resulting in the induction of a systemic inflammatory reaction. The ability of the host to adapt to this systemic inflammatory response is dependent on the magnitude of the response, the duration of the response, and the adaptive capacity of the host.

The immune system is a complicated network. Soluble mediators secreted by immune and vascular endothelial cells regulate many immune functions and serve as means of communication between different parts of the system. Mediators are involved in the regulation of their own release as well as in the production and secretion of other mediators. The existence of a network also explains why administration of a specific mediator might trigger systemic inflammation. Therapeutic intervention at different steps might be successful in the prevention of SIRS if the mediator plays a pivotal role in the development of the systemic inflammatory response. However, interventions aimed at neutralizing single mediators of SIRS such as tumor necrosis factor á (TNFá), interleukin-1 (IL-10, and platelet activating factor have in general not been successful in clinical trials. Several proinflammatory cytokines, chemokines, and non-cytokine inflammatory mediators play a role in the pathogenisis of SIRS. Cytokines comprise a broad group of polypeptides with varied functions within the immune response. The classical mediator of systemic inflammation is TNFá. TNFá is released primarily by macrophages within minutes of local or systemic injury and modulates a variety of immunologic and metabolic events. At sites of local infection or inflammation, TNFá initiates an immune response that activates antimicrobial defense mechanisms and, once the infection is eradicated, tissue repair. It is a potent activator of neutrophils and mononuclear paghocytes, and also serves as a growth factor for fibroblasts and as an angiogenesis factor. However, systemic release of TNFá can precipitate a destructive cascade of events that can result in tissue injury, organ dysfunction and, potentially, death. Among the systemic effects of TNFá are the induction of fever, stimulation of acute phase protein secretion by the liver, activation of the coagulation cascade, myocardial suppression, induction of systemic vasodilators with resultant hypotension, catabolism, and hypoglycemia. Tumor necrosis factor á is also a potent stimulus for the release of other inflammatory mediators, particularly IL-1 and IL-6. Interleukin-1 is released primarily by mononuclear phagocytes and its physiologic effects are essentially indentical to those of TNFá. However, important differences between the functions of IL-1 and TNFá exist. Most notably, IL-1 does not induce tissue injury or apoptotic cell death by itself but can potentiate the injurious effects of TNFá. Interleukin-6 is another protein that is commonly increased in the circulation of patients with SIRS. This protein is secreted by macrophages, endothelial cells, and fibroblasts. Interleukin-6 itself does not induce tissue injury but its presence in the circulation has been associated with poor outcome in trauma patients, probably because it is a marker of ongoing inflammation. Furthermore, interferon ã (IFN-ã) is a cytokine involved in the amplification of the acute inflammatory response, particularly the stimulation of cytokine secretion, phagocytosis, and respiratory burst activity by macrophages. Interferon ã is secreted primarily by T lymphocytes and natural killer (NK) cells in response to antigen presentation as well as macrophage-derived cytokines such as IL-12 and IL-18. The primary effect of IFN-ã is to amplify the inflammatory response of macrophages. In response to IFN-ã, the phagocytic and respiratory burst activity of macrophages are increased, secretion of inflammatory mediators such as TNF-á and IL-1 are enhanced, and antigen presentation is potentiated by upregulation of class II major histocompatability complex. A central feature in the upregulation of many of the soluble mediators described above is the transcription factor nuclear factor-êB (NF-êB). NF-êB is comprised of a family of proteins including p50 (NF-êB1), P65 (RelA), C-Rel, and p52 (NF-êB2) that combine to form homo- or heterodimers and ultimately function to regulate the transcription of a variety of cytokine, chemokine, adhesion molecule, and enzyme genes involved in SIRS. The binding of TNFá or IL-1â to their receptors activates a signal transduction cascade a variety of inflammation-associated gene products and modulates their expression. Increased activation of NF-êB has been associated with poor outcome in some studies. Several non-cytokine factors have been implicated in the pathogenesis of systemic inflammation. Platelet activating factor (PAF) is a phospholipid autocoid release by endothelial cells that regulates the release of cytokines and amplifies the proinflammatory response. Leukotrienes (LTC-LTE) induce contraction of endothelial cells and encourage capillary leakage. Thromboxane A, a macrophage and platelet-derived factor, promotes platelet aggregation, vasoconstriction and, potentially, tissue thrombosis. The complement cascade is comprised of more than 30 proteins that interact in a complex fashion to mediate inflammation and direct lysis of mircobes and other cells. Excessive complement activation appears to cause significant cellular injury in the host. Products of the complement cascade, most notably C3a and C5a, are potent activators of inflammation and leukocytes chemotaxis. C3a and C5a also directly activate neutrophils and promote release of reactive oxygen intermediates and proteases. Despite our increased understanding of the role of inflammatory mediators in the pathologenesis of SIRS, most anti-inflammatory drug regimes have had little success in the treatment of this problem. Neutralizing approaches to several inflammatory mediators have been studied. All of these studies have demonstrated, at best, marginal improvement in septic morbidity and mortality. One of the most widely studied approaches for the treatment of SIRS is the use of monoclonal antibodies to TNFá. Several multicenter, prospective, clinical trials have been undertaken in septic patients using several different antibodies to TNFá. These studies did not demonstrate improved outcome in patients receiving anti-TNFá compared to placebo. One recent study evaluated the efficacy of a chimeric antibody to TNF in patients with severe sepsis.

Circulating levels of TNFá as well as a variety of other inflammatory mediators were assessed. Although circulating levels of TNFá were transiently decreased, anti-TNFá therapy did not result in reduction of circulating levels of other inflammatory mediators such as IL-1â, IL-1ra, sTNFR, or IL-6. In addition, evidence of systemic inflammation was not decreased and overall mortality was not improved in anti-TNFá treated patients. Because the relative ineffectiveness of anti-inflammatory therapy aimed at neutralizing single mediators, more broad-based strategies with the goal of neutralizing, removing, or inhibiting the production of several inflammatory mediators are looked forward to. The use of glucocorticoids in the treatment of sepsis has been proposed for more than 30 years. Overall, the use of glucocorticoids to treat sepsis and septic shock has not been beneficial. In many studies, the use of glucocorticoids in septic patients was associated with increased mortality. In burned patients, there is no evidence that administration of glucocorticoids provides effective treatment for systemic inflammation.

Reasons for the lack of efficacy of these agents are likely to be multifactorial. Firstly, the inflammatory response to injury and spesis is mediated by a complex array of mediators that are largely interrelated. Therefore, blocking or neutralization of a single mediator is not likely to have a marked effect on the overall response. Secondly, the same mediators that are important in inducing tissue injury also play an important role in antimicrobial immunity. Blockade of these mediators may leave the host more susceptible to subsequent infection. Thirdly, many of the mediators, particulary TNFá and IL-1â, are released within minutes of the injury and mobilize the inflammatory cascade shortly thereafter.

Paradoxically, a state of immunosuppression often follows or co-exists with SIRS. The counter anti-inflammatory response syndrome (CARS) appears to be an adaptive mechanism designed to limit the injurious effects of systemic inflammation. However, this response may also render the host more susceptible to systemic infection due to impaired antimicrobial immunity. CARS is often seen after serious trauma. Virtually all components of the immune response have been found to be depressed following injury including macrophage, lymphocyte, and neutrophil function; delayed type hypersensitivity (DTH) responses, immunoglobulin (Ig) and interferon (IFN) production, and serum opsonic capacity. Serum peptides, which suppress lymphocyte proliferation *in vitro*, have been defined, and the immunosuppressive role of excessive complement activation has also been recognized. Immune failure occurs early after trauma and the rapidity with which immune function returns to normal may be the best indicator of clinical recovery. Indeed, immediate down-regulation of the immune response may be a protective mechanism for the host, lest too vigorous an early host response creates a catabolic situation incompatible with early survival. The surface expression of the class II HLA-DR on peripheral blood monocytes was measured in 60 patients and was depressed in most, immediately following severe trauma and during subsequent sepsis. However, when patients were grouped according to clinical outcome (uneventful recovery, major infection, and death) an interesting pattern arose. The percentage of monocytes that expressed the HLA-DR antigen returned to the normal range by one week in the first group, by three weeks in those with major infection, but never in those who eventually died. Thus, antigen expression served as a useful marker, or predictor, of clinical outcome in such patients. When monocytes were incubated with bacterial lipopolysaccharide (LPS), those patients who survived had enhanced HLA-DR antigen expression (stimulated towards the normal range), while monocytes from patients who died were relatively resistant to stimulation. Expression of HLA-DR antigen correlates with the ability of these cells to present foreign antigen and thus initiate a specific immune response.

The invention provides a method for treating a subject suspected to suffer from a disease comprising an inflammatory condition and/or a counter-inflammatory condition comprising subjecting said subject to a diagnostic process aimed at determining inflammatory disease stage of said subject further comprising providing said subject with a gene-regulatory peptide or functional analogue thereof depending on the outcome of said determination of disease stage. In one embodiment said diagnostic process includes determining the level of a pro-inflammatory cytokine, such as pro-inflammatory cytokines that are responsible for early responses are IL1-alpha, IL1-beta, IL6, and TNF-alpha. Other pro-inflammatory mediators include LIF, IFN-gamma, OSM, CNTF, GM-CSF, IL11, IL12, IL17, IL18, IL8 and a variety of other chemokines that chemoattract inflammatory cells, and various neuromodulatory factors. It is preferred that said diagnostic process includes determining the level of a pro-inflammatory cytokine that is selected from the group of tumor necrosis factor-alpha, interferon-gamma, interleukin-1-beta and interleukin-6. Upon determination of pro-inflammatory cytokine levels, and having determined the disease stage as an essentially inflammatory condition, it is herein also provided to treat said subject with a gene-regulatory peptide or functional analogue that down-regulates translocation and/or activity of pro-inflammatory cytokine gene expression mediated by a gene transcription factor. It is for example useful, when said gene transcription factor comprises an NF-kappaB/Rel protein, to inhibit translocation and/or activity of said NF-kappaB/Rel protein. Such inhibition is preferably achieved with a gene-regulatory peptide selected from the group of peptides having NFkappaB down-regulating activity in LPS stimulated RAW264.7 cells.

In another embodiment, said diagnostic process includes determining the level of a counter-inflammatory cytokine which may however be combined with determining for example TNF-alpha levels, to corroborated the diagnostic process. In one embodiment said diagnostic process includes determining the level of a counter-inflammatory cytokine, such as for example IL4, IL10, and IL13, IL16, IFN-alpha, IL1ra, G-CSF, soluble receptors for TNF or IL6. It is preferred that said counter-inflammatory cytokine is selected from the group of interleukin-4 and interleukin-10.

An essentially inflammatory condition is preferably characterized by elevated levels of at least one, but preferably at least two or three pro-inflammatory cytokines for example produced by circulating polymorph bone marrow cells (PBMCs), such as elevated plasma or serum levels of one or more of the pro-inflammatory cytokines that are responsible for early responses such as IL1-alpha, IL1-beta, IL6, and TNF-alpha. Other pro-inflammatory mediators include LIF, IFN-gamma, OSM, CNTF, GM-CSF, IL11, IL12, IL17, IL18, IL8. As each diagnostician may have his or her own preference for testing an inflammatory mediator, as said, it is preferred that said diagnostic process is based on determining the level of a pro-inflammatory cytokine that is selected from the group of tumor necrosis factor-alpha, interferon-gamma, interleukin-1-beta and interleukin-6. PBMCs of patients in an essentially inflammatory condition in general produce plasma levels of at least >2 SD above the control mean (CM) values of TNF-alpha, IL-1beta, and/or IL-6 produced by control PBMCs from non-diseased individuals of comparable age and background. However, testing other pro-inflammatory mediators in other test samples may be preferred by the individual diagnostician based on his or her own experience and preferences.

An essentially counter-inflammatory condition is preferably characterized by elevated levels of at least one, but preferably at least two or three counter-inflammatory cytokines produced by circulating PBMCs, such as elevated plasma levels of one or more of the a counter-inflammatory cytokine, such as for example IL4, IL10, and IL13, IL16, IFN-alpha, IL1ra, G-CSF, soluble receptors for TNF or IL6. It is preferred that said counter-inflammatory cytokine is selected from the group of interleukin-4 and interleukin-10. PBMCs of patients in an essentially counter-inflammatory condition in general produce at least >2 SD above the control mean (CM) values of IL-4 or IL-10 produced by control PBMCs from non-diseased individuals of comparable age and background. However, testing other counter-inflammatory mediators in other test samples may be preferred by the individual diagnostician based on his or her own experience and preferences.

To validate a diagnosis of an essentially inflammatory condition, it may be useful in the diagnostic process to also determine the levels of one or more counter-inflammatory mediators, and vice-versa. Useful are diagnostic tests such as flow cytometry assays of serum/plasma/supernatant available with BD Biosciences as a Cytometric Bead Array (CBA), for example a CBA human Th1/Th2 cytokine kit for the measurement of IL-2, IL-4, IL-5, IL-10, TNF-a and IFN-g in a single samplr or available with Biosource International, the Biosource Multiplex antibody Bead Kit for measurement of IL-1b, IL-2, IL-4, IL-5, IL-6, IL-8, IL-10, IFN-g, TNF-a and GM-CSF in single sample or for example the Biosource cytoset ELISA for measurement of individual cytokines and soluble DR4. Inflammation mediators could be also measured by HPLC with the help of FITC labeled specific antibodies.

It is herein also provide to use a diagnostic process that includes determining HLA-DR expression on circulating monocytes of said subject. HLA-DR antigen under expression indicates a counter-inflammatory condition and is a discriminator of poor clinical outcome. Such a patient is then treated with a gene-regulatory peptide that up-regulates pro-inflammatory cytokine gene expression. Such a peptide is preferably selected from the group of peptides having NFkappaB up-regulating activity in LPS stimulated RAW264.7 cells.

In another embodiment, the invention provides using a diagnostic process that includes determining arachidonic acid metabolite levels in said subject or a diagnostic process that includes plasma prostaglandin levels in said subject. In particular, determining a ratio between prostaglandins 1 and 2 (PGE1 and PGE2) is useful, whereby a high PGE2 levels are indicative for an inflammatory condition which should be treated accordingly.

In short, the invention provides a method of treatment of a subject suspected to suffer from a disease comprising an inflammatory condition and/or a counter-inflammatory condition whereby it is provided to treat said subject with a gene-regulatory peptide or functional analogue thereof capable of down-regulation of inflammation after having determined disease stage as an essentially inflammatory condition. A subject with an essentially inflammatory condition is preferably treated with a gene-regulatory peptide that down-regulates translocation and/or activity of pro-inflammatory cytokine gene expression mediated by a gene transcription factor. It is preferred that said gene transcription factor comprises an NF-kappaB/Rel protein whereby translocation and/or activity of said NF-kappaB/Rel protein is inhibited by said peptide. Such a peptide is preferably selected from the group of peptides having NFkappaB down-regulating activity in LPS stimulated RAW264.7 cells, and even more preferably from the group of peptides having NFkappaB down-regulating activity in LPS unstimulated RAW264.7 cells.

Also, the invention provides a method of treatment of a subject suspected to suffer from a disease comprising an inflammatory condition and/or a counter-inflammatory condition whereby it is provided to treat said subject with a gene-regulatory peptide or functional analogue thereof capable of up-regulation of inflammation after having determined disease stage as an essentially counter-inflammatory condition. A subject with an essentially counter inflammatory condition is preferably treated with a gene-regulatory peptide that up-regulates translocation and/or activity of pro-inflammatory cytokine gene expression mediated by a gene transcription factor. In one embodiment, it is preferred to treat said counter-inflammatory condition with a gene-regulatory peptide that activates an NF-kappaB/Rel protein. Than it is preferred that translocation and/or activity of said NF-kappaB/Rel protein is activated. Such a peptide is preferably selected from the group of peptides having NFkappaB up-regulating activity in LPS unstimulated RAW264.7 cells, more preferably from the group of peptides having NFkappaB up-regulating activity in LPS stimulated RAW264.7 cells.

The invention also provides use of a gene-regulatory peptide or functional analogue thereof for the production of a pharmaceutical composition for the treatment of a disease comprising an inflammatory condition and/or a counter-inflammatory condition wherein a subject suffering from said disease is subjected to a diagnostic process aimed at determining inflammatory disease stage of said subject and where treatment is selected depending on the outcome of said determination of disease stage, for example wherein said diagnostic process includes determining the level of a pro-inflammatory cytokine and said pro-inflammatory cytokine is selected from the group of tumor necrosis factor-alpha, interferon-gamma, interleukin-1-beta and interleukin-6.

When said diagnostic process includes determining the level of a counter-inflammatory cytokine it is preferred that counter-inflammatory cytokine is selected from the group of interleukin-4 and interleukin-10.

The invention provides treating said subject with a gene-regulatory peptide or functional analogue thereof based on the determination of disease stage as an essentially inflammatory condition. It is than preferred to treat the subject with a gene-regulatory peptide down-regulates translocation and/or activity of pro-inflammatory cytokine gene expression mediated by a gene transcription factor, preferably selected from the group of peptides or analogues having NFkappaB down-regulating activity in LPS stimulated RAW264.7 cells.

On the other hand, when treatment is selected on the basis of the determination of disease stage as an essentially counter-inflammatory condition, said treatment preferably is done with a gene-regulatory peptide that up-regulates translocation and/or activity of pro-inflammatory cytokine gene expression mediated by a gene transcription factor, such as with a peptide is selected from the group of peptides having NFkappaB up-regulating activity in LPS un-stimulated RAW264.7 cells.

The treatment of sepsis/SIRS, as is for example provided herein, is in one embodiment directed at inhibiting the production and release of immune mediators involved in the generation of sepsis/SIRS, thereby blocking the upward spiral of SIRS. Inhibiting the production of these mediators is achieved by regulating particular gene transcription activators with a gene-regulatory peptide as provided herein. A particular family of gene transcription activators, generally and widely known to be central in the activation of genes leading to the production of immunological mediators, is the NF-kappaB protein family. The ability to inhibit the NF-kappaB protein family is currently a widely sought after *desideratum* for the development of immunomodulating therapeutic approaches, the family being so central in shaping a wide array of immune responses of the body. Gene-regulatory peptides as herein have the ability to inhibit proteins of this family.

### Examples

In a particular embodiment, the invention relates to treatment of the systemic inflammatory response seen with sepsis/SIRS/CARS and caused by said immune mediators, said treatment comprising inhibiting the production and effects of said mediators by inhibiting gene expression, in particular by inhibiting gene expression regulated via the NF-kappaB protein family. It is also realized that defining patients at very high risk of infection and multi-system organ failure *before* either develop, is paramount to the introduction and interpretation of clinical treatment in this area.

A gene regulatory peptide is preferably a short peptide, preferably of at most 30 amino acids long, or a functional analogue or derivative thereof. In a much preferred embodiment, the peptide is from about 3 to about 15 amino acids long, preferably 4 to 12, more preferably 4 to 9, most preferably 4 to 6 amino acids long, or a functional analogue or derivative thereof. Of course, such a gene-regulatory peptide can be longer, for example by extending it (N- and/or C-terminally), with more amino acids or other side groups, which can for example be (enzymatically) cleaved off when the molecule enters the place of final destination. In particular a method is provided wherein said gene-regulatory peptide modulates translocation and/or activity of a gene transcription factor. It is particularly useful when said gene transcription factor comprises an NF-kappaB/Rel protein or an AP-1 protein. Insults generally induce increased expression of inflammatory cytokines due to activation of NF-κB and AP-1, and in a preferred embodiment the invention provides a method wherein translocation and/or activity of said NF-kappaB/Rel protein is inhibited. In one embodiment, said peptide is selected from the group of peptides LQG, AQG, LQGV, AQGV, LQGA, VLPALP, ALPALP, VAPALP, ALPALPQ, VLPAAPQ, VLPALAQ, LAGV, VLAALP, VLAALP, VLPALA, VLPALPQ, VLAALPQ, VLPALPA, GVLPALP, LQGVLPALPQVVC, LPGCPRGVNPVVS, LPGC, MTRV, MTR, VVC. Insults often induce increased expression of inflammatory cytokines due to activation of NF-κB and AP-1. Inflammatory cytokines can be expressed by epithelium, perivascular cells and adherent or transmigrating leukocytes, inducing numerous pro-inflammatory and procoagulant effects. Together these effects predispose to inflammation, thrombosis and hemorrhage. Of clinical and medical interest and value, the present invention provides the opportunity to selectively control NFκB-dependent gene expression in tissues and organs in a living subject, preferably in a primate, allowing upregulating essentially anti-inflammatory responses such as IL-10, and downregulating essentially pro-inflammatory responses such as mediated by TNF-alpha, nitric oxide (NO), IL-5, IL-1beta. The invention thus provides use of a NFκB regulating peptide or derivative thereof for the production of a pharmaceutical composition for the treatment of an inflammatory condition, preferably in a primate, and provides a method of treatment of an inflammatory condition, notably in a primate. It is preferred when the treatment directed against an inflammatory condition comprises administering to the subject a pharmaceutical composition comprising an NFkappaB down-regulating peptide or functional analogue thereof. Examples of useful NFkappaB down-regulating peptides are VLPALPQVVC, LQGVLPALPQ, LQG, LQGV, GVLPALPQ, VLPALP, VVC, MTR and circular LQGVLPALPQVVC. More down-regulating peptides and functional analogues can be found using the methods as provided herein. Most prominent among NFkappaB down-regulating peptides are VLPALPQVVC, LQGVLPALPQ, LQG, LQGV, and VLPALP. These are also capable of reducing production of NO by a cell. It is herein also provided to use a composition that comprises at least two oligopeptides or functional analogues thereof, each capable of down-regulation NFkappaB, and thereby reducing production of NO and/or TNF-alpha by a cell, in particular wherein the at least two oligopeptides are selected from the group LQGV, AQGV and VLPALP, for the treatment of inflammatory condition, and, moreover to treat the systemic inflammatory response often seen in severe burn patients.

The invention thus provides use of a NFκB regulating peptide or derivative thereof for the production of a pharmaceutical composition for the treatment of a subject suffering from a counter-inflammatory condition, in particular of a human, and provides a method of treatment of a counter-inflammatory condition. It is preferred when the treatment comprises administering to the subject a pharmaceutical composition comprising an NFkappaB up-regulating peptide or functional analogue thereof. The invention for this purpose provides use of a such signaling molecule comprising a NF-kappaB up-regulating peptide or functional analogue thereof for the production of a pharmaceutical composition for the treatment of a counter anti-inflammatory condition, for example occurring after a traumatic injury of a subject, in particular wherein translocation and/or activity of said NF-kappaB/Rel protein is upregulated, resulting in stimulating a cascade of cytokine reactions. In one embodiment, the invention is providing a method and means to treat the systemic immunosuppressive reaction by providing a subject believed to be in need thereof with a pharmaceutical composition comprising a NF-kappaB up-regulating peptide or functional analogue thereof, preferably an NFkappaB regulating peptide such as VLPALPQ, GVLPALP or MTRV, or mixtures of two or three of these peptides.

A gene-regulatory peptide such as an NFkappaB regulating peptide can be given by infusion, the peptide (or analogue) concentration preferably being from about 1 to about 1000mg/l, but the peptide can also been given in a bolus injection. Doses of 1 to 5 mg/kg bodyweight, for example every eight hours in a bolus injection or by infusion until the patient stabilizes, are recommended. It is preferred to monitor cytokine profiles, such as TNF-alpha or IL-10 levels, in the plasma of the treated patient, and to stop treatment when these levels are considered within normal boundaries.

More gene-regulating peptides and functional analogues can be found in a (bio)assay, such as a NFkappaB translocation assay as provided herein, and a by testing peptides for NFkappaB down- or up-regulating activity in LPS-stimulated or unstimulated RAW264.7 cells. For anti-inflammatory treatment, it is preferred that said peptide is selected from the group of peptides having NFkappaB down-regulating activity in LPS stimulated RAW264.7 cells, especially when said subject is at risk to experience SIRS. For treatment of an immunosuppressed state, such as seen in CARS, it is preferred that said peptide is selected from the group of peptides having NFkappaB up-regulating activity in LPS stimulated RAW264.7 cells, especially when said subject is at risk to experience CARS.

In response to a variety of pathophysiological and developmental signals, the NFkB/Rel family of transcription factors are activated and form different types of hetero- and homodimers among themselves to regulate the expression of target genes containing kappaB-specific binding sites. NF-kB transcription factors are hetero- or homodimers of a family of related proteins characterized by the Rel homology domain. They form two subfamilies, those containing activation domains (p65-RELA, RELB, and c-REL) and those lacking activation domains (p50, p52). The prototypical NFkB is a heterodimer of p65 (RELA) and p50 (NF-kB1). Among the activated NFkB dimers, p50-p65 heterodimers are known to be involved in enhancing the transcription of target genes and p50-p50 homodimers in transcriptional repression. However, p65-p65 homodimers are known for both transcriptional activation and repressive activity against target genes. KappaB DNA binding sites with varied affinities to different NFB dimers have been discovered in the promoters of several eukaryotic genes and the balance between activated NFkB homo- and heterodimers ultimately determines the nature and level of gene expression within the cell. The term "NFkB-regulating peptide" as used herein refers to a peptide or a modification or derivative thereof capable of modulating the activation of members of the NFkB/Rel family of transcription factors. Activation of NFkB can lead to enhanced transcription of target genes. Also, it can lead to transcriptional repression of target genes. NFkB activation can be regulated at multiple levels. For example, the dynamic shuttling of the inactive NFkB dimers between the cytoplasm and nucleus by IkappaB proteins and its termination by phosphorylation and proteasomal degradation, direct phosphorylation, acetylation of NFkB factors, and dynamic reorganization of NFkB subunits among the activated NFkB dimers have all been identified as key regulatory steps in NFkB activation and, consequently, in NFkB-mediated transcription processes. Thus, an NFkB-regulating peptide is capable of modulating the transcription of genes that are under the control of NFkB/Rel family of transcription factors. Modulating comprises the upregulation or the downregulation of transcription. In a preferred embodiment, a peptide according to the invention, or a functional derivative or analogue thereof is used for the production of a pharmaceutical composition. Such peptides are preferably selected from group of peptides having NFkappaB down-regulating activity in LPS stimulated RAW264.7 cells. Examples of useful NFkappaB down-regulating peptides to be included in such a pharmaceutical composition are VLPALPQVVC, LQGVLPALPQ, LQG, LQGV, GVLPALPQ, VLPALP, VVC, MTR and circular LQGVLPALPQVVC. More gene-regulating peptides and functional analogues can be found in a (bio)assay, such as a NFkappaB translocation assay as provided herein, which can also be used to further identify peptides having NFkappaB up-regulating activity in LPS stimulated RAW264.7 cells. Most prominent among NFkappaB down-regulating peptides are VLPALPQVVC, LQGVLPALPQ, LQG, LQGV, and VLPALP. These are also capable of reducing production of NO by a cell. It is herein also provided to use a composition that comprises at least two oligopeptides or functional analogues thereof, each capable of down-regulation NFkappaB, and thereby reducing production of NO and/or TNF-alpha by a cell, in particular wherein the at least two oligopeptides are selected from the group LQGV, AQGV and VLPALP. Useful NFkappaB up-regulating peptides are VLPALPQ, GVLPALP and MTRV. As indicated, more gene-regulatory peptides may be found with an appropriate (bio)assay. A gene-regulatory peptide as used herein is preferably short. Preferably, such a peptide is 3 to 15 amino acids long, more preferably, wherein said lead peptide is 3 to 9 amino acids long, most preferred wherein said lead peptide is 4 to 6 amino acids long, and capable of modulating the expression of a gene, such as a cytokine, in a cell. In a preferred embodiment, a peptide is a gene-regulatory peptide that is capable of traversing the plasma membrane of a cell or, in other words, a peptide that is membrane-permeable.

Functional derivative or analogue herein relates to the signaling molecular effect or activity as for example can be measured by measuring nuclear translocation of a relevant transcription factor, such as NF-kappaB in an NF-kappaB assay, or AP-1 in an AP-1 assay, or by another method as provided herein. Fragments can be somewhat (i.e. 1 or 2 amino acids) smaller or larger on one or both sides, while still providing functional activity. Such a bioassay comprises an assay for obtaining information about the capacity or tendency of a peptide, or a modification thereof, to regulate expression of a gene. A scan with for example a 15-mer, or a 12-mer, or a 9-mer, or a 8-mer, or a 7-mer, or a 6-mer, or a 5-mer, or a 4-mer or a 3-mer peptides can yield valuable information on the linear stretch of amino acids that form an interaction site and allows identification of gene-regulatory peptides that have the capacity or tendency to regulate gene expression. Gene-regulatory peptides can be modified to modulate their capacity or tendency to regulate gene expression, which can be easily assayed in an in vitro bioassay such as a reporter assay. For example, some amino acid at some position can be replaced with another amino acid of similar or different properties. Alanine (Ala)-replacement scanning, involving a systematic replacement of each amino acid by an Ala residue, is a suitable approach to modify the amino acid composition of a gene-regulatory peptide when in a search for a gene-regulatory peptide capable of modulating gene expression. Of course, such replacement scanning or mapping can be undertaken with amino acids other than Ala as well, for example with D-amino acids. In one embodiment, a peptide derived from a naturally occurring polypeptide is identified as being capable of modulating gene expression of a gene in a cell. Subsequently, various synthetic Ala-mutants of this gene-regulatory peptide are produced. These Ala-mutants are screened for their enhanced or improved capacity to regulate expression of a gene compared to gene-regulatory polypeptide.

Furthermore, a gene-regulatory peptide, or a modification or analogue thereof, can be chemically synthesised using D- and / or L-stereoisomers. For example, a gene-regulatory peptide that is a retro-inverso of an oligopeptide of natural origin is produced. The concept of polypeptide retro-inversion (assemblage of a natural L-amino acid-containing parent sequence in reverse order using D-amino acids) has been applied successfully to synthetic peptides. Retro-inverso modification of peptide bonds has evolved into a widely used peptidomimetic approach for the design of novel bioactive molecules which has been applied to many families of biologically active peptide. The sequence, amino acid composition and length of a peptide will influence whether correct assembly and purification are feasible. These factors also determine the solubility of the final product. The purity of a crude peptide typically decreases as the length increases. The yield of peptide for sequences less than 15 residues is usually satisfactory, and such peptides can typically be made without difficulty. The overall amino acid composition of a peptide is an important design variable. A peptide's solubility is strongly influenced by composition. Peptides with a high content of hydrophobic residues, such as Leu, Val, Ile, Met, Phe and Trp, will either have limited solubility in aqueous solution or be completely insoluble. Under these conditions, it can be difficult to use the peptide in experiments, and it may be difficult to purify the peptide if necessary. To achieve a good solubility, it is advisable to keep the hydrophobic amino acid content below 50% and to make sure that there is at least one charged residue for every five amino acids. At physiological pH Asp, Glu, Lys, and Arg all have charged side chains. A single conservative replacement, such as replacing Ala with Gly, or adding a set of polar residues to the N- or C-terminus, may also improve solubility. Peptides containing multiple Cys, Met, or Trp residues can also be difficult to obtain in high purity partly because these residues are susceptible to oxidation and/or side reactions. If possible, one should choose sequences to minimize these residues. Alternatively, conservative replacements can be made for some residues. For instance, Norleucine can be used as a replacement for Met, and Ser is sometimes used as a less reactive replacement for Cys. If a number of sequential or overlapping peptides from a protein sequence are to be made, making a change in the starting point of each peptide may create a better balance between hydrophilic and hydrophobic residues. A change in the number of Cys, Met, and Trp residues contained in individual peptides may produce a similar effect. In another embodiment of the invention, a gene-regulatory peptide capable of modulating gene expression is a chemically modified peptide. A peptide modification includes phosphorylation (e.g on a Tyr, Ser or Thr residue), N-terminal acetylation, C-terminal amidation, C-terminal hydrazide, C-terminal methyl ester, fatty acid attachment, sulfonation (tyrosine), N-terminal dansylation, N-terminal succinylation, tripalmitoyl-S-Glyceryl Cysteine (PAM3 Cys-OH) as well as farnesylation of a Cys residue. Systematic chemical modification of a gene-regulatory peptide can for example be performed in the process of gene-regulatory peptide optimalization.

Synthetic peptides can be obtained using various procedures known in the art. These include solid phase peptide synthesis (SPPS) and solution phase organic synthesis (SPOS) technologies. SPPS is a quick and easy approach to synthesize peptides and small proteins. The C-terminal amino acid is typically attached to a cross-linked polystyrene resin via an acid labile bond with a linker molecule. This resin is insoluble in the solvents used for synthesis, making it relatively simple and fast to wash away excess reagents and by-products.

The peptides as mentioned in this document such as LQG, AQG, LQGV, AQGV, LQGA, VLPALP, ALPALP, VAPALP, ALPALPQ, VLPAAPQ, VLPALAQ, LAGV, VLAALP, VLPALA, VLPALPQ, VLAALPQ, VLPALPA, GVLPALP, VVCNYRDVRFESIRLPGCPRGVNPVVSYAVALSCQCAL, RPRCRPINATLAVEKEGCPVCITVNTTICAGYCPT, SKAPPPSLPSPSRLPGPS, LQGVLPALPQVVC, SIRLPGCPRGVNPVVS, LPGCPRGVNPVVS, LPGC, MTRV, MTR, and VVC were prepared by solid-phase synthesis using the fluorenylmethoxycarbonyl (Fmoc)/tert-butyl-based methodology with 2-chlorotrityl chloride resin as the solid support. The side-chain of glutamine was protected with a trityl function. The peptides were synthesized manually. Each coupling consisted of the following steps: (i) removal of the alpha-amino Fmoc-protection by piperidine in dimethylformamide (DMF), (ii) coupling of the Fmoc amino acid (3 eq) with diisopropylcarbodiimide (DIC)/1-hydroxybenzotriazole (HOBt) in DMF/N-methylformamide (NMP) and (iii) capping of the remaining amino functions with acetic anhydride/diisopropylethylamine (DIEA) in DMF/NMP. Upon completion of the synthesis, the peptide resin was treated with a mixture of trifluoroacetic acid (TFA)/H₂O/triisopropylsilane (TIS) 95:2.5:2.5. After 30 minutes TIS was added until decolorization. The solution was evaporated in vacuo and the peptide precipitated with diethylether. The crude peptides were dissolved in water (50-100 mg/ml) and purified by reverse-phase high-performance liquid chromatography (RP-HPLC). HPLC conditions were: column: Vydac TP21810C18 (10 x 250 mm); elution system: gradient system of 0.1% TFA in water v/v (A) and 0.1% TFA in acetonitrile (ACN) v/v (B); flow rate 6 ml/min; absorbance was detected from 190-370 nm. There were different gradient systems used. For example for peptides LQG and LQGV: 10 minutes 100% A followed by linear gradient 0-10% B in 50 minutes. For example for peptides VLPALP and VLPALPQ: 5 minutes 5% B followed by linear gradient 1% B/minute. The collected fractions were concentrated to about 5 ml by rotation film evaporation under reduced pressure at 40°C. The remaining TFA was exchanged against acetate by eluting two times over a column with anion exchange resin (Merck II) in acetate form. The elute was concentrated and lyophilised in 28 hours. Peptides later were prepared for use by dissolving them in PBS.

RAW 264.7 macrophages, obtained from American Type Culture Collection (Manassas, VA), were cultured at 37°C in 5% CO2 using DMEM containing 10% FBS and antibiotics (100 U/ml of penicillin, and 100 µg/ml streptomycin). Cells (1 x10⁶/ml) were incubated with peptide (10 µg/ml) in a volume of 2 ml. After 8 h of cultures cells were washed and prepared for nuclear extracts.

Nuclear extracts and EMSA were prepared according to Schreiber et al. Methods (Schreiber et al. 1989, Nucleic Acids Research 17). Briefly, nuclear extracts from peptide stimulated or nonstimulated macrophages were prepared by cell lysis followed by nuclear lysis. Cells were then suspended in 400 µl of buffer (10 mM HEPES (pH 7.9), 10 mM KCl, 0.1 mM KCL, 0.1 mM EDTA, 0.1 mM EGTA, 1 mM DTT, 0.5 mM PMSF and protease inhibitors), vigorously vortexed for 15 s, left standing at 4°C for 15 min, and centrifuged at 15,000 rpm for 2 min. The pelleted nuclei were resuspended in buffer (20 mM HEPES (pH 7.9), 10% glycerol, 400 mM NaCl, 1 mM EDTA, 1mM EGTA, 1 mM DTT, 0.5 mM PMSF and protease inhibitors) for 30 min on ice, then the lysates were centrifuged at 15,000 rpm for 2 min. The supernatants containing the solubilized nuclear proteins were stored at -70°C until used for the Electrophoretic Mobility Shift Assays (EMSA).

Electrophoretic mobility shift assays were performed by incubating nuclear extracts prepared from control (RAW 264.7) and peptide treated RAW 264.7 cells with a 32P-labeled double-stranded probe (5' AGCTCAGAGGGGGACTTTCCGAGAG 3') synthesized to represent the NF-kappaB binding sequence. Shortly, the probe was end-labeled with T4 polynucleotide kinase according to manufacturer's instructions (Promega, Madison, WI). The annealed probe was incubated with nuclear extract as follows: in EMSA, binding reaction mixtures (20 µl) contained 0.25 µg of poly(dI-dC) (Amersham Pharmacia Biotech) and 20,000 rpm of 32P-labeled DNA probe in binding buffer consisting of 5 mM EDTA, 20% Ficoll, 5 mM DTT, 300 mM KCl and 50 mM HEPES. The binding reaction was started by the addition of cell extracts (10 µg) and was continued for 30 min at room temperature. The DNA-protein complex was resolved from free oligonucleotide by electrophoresis in a 6% polyacrylamide gel. The gels were dried and exposed to x-ray films.

The transcription factor NF-kB participates in the transcriptional regulation of a variety of genes. Nuclear protein extracts were prepared from LPS and peptide treated RAW264.7 cells or from LPS treated RAW264.7 cells. In order to determine whether the peptide modulates the translocation of NF-kB into the nucleus, on these extracts EMSA was performed. Here we see that indeed some peptides are able to modulate the translocation of NF-kB since the amount of labeled oligonucleotide for NF-kB is reduced. In this experiment peptides that show the modulation of translocation of NF-kB are: VLPALPQVVC, LQGVLPALPQ, LQG, LQGV, GVLPALPQ, VLPALP, VLPALPQ, GVLPALP, VVC, MTRV, MTR.

RAW 264.7 mouse macrophages were cultured in DMEM, containing 10% or 2% FBS, penicillin, streptomycin and glutamine, at 37 °C, 5% CO₂. Cells were seeded in a 12-wells plate (3x10⁶ cells/ml) in a total volume of 1 ml for 2hours and then stimulated with LPS (E. coli 026:B6; Difco Laboratories, Detroit, MI, USA) and/or peptide (1 microgr/ml). After 30 minutes of incubation plates were centrifuged and cells were collected for nuclear extracts. Nuclear extracts and EMSA were prepared according to Schreiber et al. Cells were collected in a tube and centrifuged for 5 minutes at 2000 rpm (rounds per minute) at 4°C (Universal 30 RF, Hettich Zentrifuges). The pellet was washed with ice-cold Tris buffered saline (TBS pH 7.4) and resuspended in 400 µl of a hypotonic buffer A (10 mM HEPES pH 7.9, 10 mM KCl, 0.1 mM EDTA, 0.1 mM EGTA, 1 mM DTT, 0.5 mM PMSF and protease inhibitor cocktail (Complete™ Mini, Roche) and left on ice for 15 minutes. Twenty five micro litre 10% NP-40 was added and the sample was centrifuged (2 minutes, 4000 rpm, 4°C). The supernatant (cytoplasmic fraction) was collected and stored at -70°C. The pellet, which contains the nuclei, was washed with 50 µl buffer A and resuspended in 50 µl buffer C (20 mM HEPES pH 7.9, 400 mM NaCl, 1 mM EDTA, 1 mM EGTA, 1 mM DTT, 0.5 mM PMSF and protease inhibitor cocktail and 10% glycerol). The samples were left to shake at 4°C for at least 60 minutes. Finally the samples were centrifuged and the supernatant (nucleic fraction) was stored at -70°C.

Bradford reagent (Sigma) was used to determine the final protein concentration in the extracts. For Electrophoretic mobility shift assays an oligonucleotide representing NF-κB binding sequence (5'-AGC TCA GAG GGG GAC TTT CCG AGA G-3') was synthesized. Hundred pico mol sense and antisense oligo were annealed and labelled with γ-³²P-dATP using T4 polynucleotide kinase according to manufacture's instructions (Promega, Madison, WI). Nuclear extract (5-7.5 µg) was incubated for 30 minutes with 75000 cpm probe in binding reaction mixture (20 microliter) containing 0.5 µg poly dI-dC (Amersham Pharmacia Biotech) and binding buffer BSB (25 mM MgCl₂, 5 mM CaCl₂, 5mM DTT and 20% Ficoll) at room temperature. The DNA-protein complex was resolved from free oligonucleotide by electrophoresis in a 4-6% polyacrylamide gel (150 V, 2-4 hours). The gel was then dried and exposed to x-ray film. The transcription factor NF-kB participates in the transcriptional regulation of a variety of genes. Nuclear protein extracts were prepared from either LPS (1 mg/ml), peptide (1 mg/ml) or LPS in combination with peptide treated and untreated RAW264.7 cells. In order to determine whether the peptides modulate the translocation of NF-kB into the nucleus, on these extracts EMSA was performed. Peptides are able to modulate the basal as well as LPS induced levels of NF-kB. In this experiment peptides that show the inhibition of LPS induced translocation of NF-kB are: VLPALPQVVC, LQGVLPALPQ, LQG, LQGV, GVLPALPQ, VLPALP, VVC, MTR and circular LQGVLPALPQVVC. Peptides that in this experiment promote LPS induced translocation of NF-kB are: VLPALPQ, GVLPALP and MTRV. Basal levels of NF-kB in the nucleus was decreased by VLPALPQVVC, LQGVLPALPQ, LQG and LQGV while basal levels of NF-kB in the nucleus was increased by GVLPALPQ, VLPALPQ, GVLPALP, VVC, MTRV, MTR and LQGVLPALPQVVC. In other experiments, QVVC also showed the modulation of translocation of NF-kB into nucleus (data not shown).

Further modes of identification of gene-regulatory peptides by NFkB analysis

*Cells:* Cells will be cultured in appropriate culture medium at 37 °C, 5% CO₂. Cells will be seeded in a 12-wells plate (usually 1x10⁶ cells/ml) in a total volume of 1 ml for 2hours and then stimulated with regulatory peptide in the presence or absence of additional stimuli such as LPS. After 30 minutes of incubation plates will be centrifuged and cells are collected for cytosolic or nuclear extracts.

Nuclear Extracts: Nuclear extracts and EMSA could be prepared according to Schreiber et al. Method (Schriber et al. 1989, Nucleic Acids Research 17). Cells are collected in a tube and centrifuged for 5 minutes at 2000 rpm (rounds per minute) at 4°C (Universal 30 RF, Hettich Zentrifuges). The pellet is washed with ice-cold Tris buffered saline (TBS pH 7.4) and resuspended in 400 µl of a hypotonic buffer A (10 mM HEPES pH 7.9, 10 mM KCl, 0.1 mM EDTA, 0.1 mM EGTA, 1 mM DTT, 0.5 mM PMSF and protease inhibitor cocktail (Complete™ Mini, Roche) and left on ice for 15 minutes. Twenty five micro litre 10% NP-40 is added and the sample is centrifuged (2 minutes, 4000 rpm, 4°C). The supernatant (cytoplasmic fraction) was collected and stored at -70°C for analysis. The pellet, which contains the nuclei, is washed with 50 µl buffer A and resuspended in 50 µl buffer C (20 mM HEPES pH 7.9, 400 mM NaCl, 1 mM EDTA, 1 mM EGTA, 1 mM DTT, 0.5 mM PMSF and protease inhibitor cocktail and 10% glycerol). The samples are left to shake at 4°C for at least 60 minutes. Finally the samples are centrifuged and the supernatant (nucleic fraction) is stored at -70°C for analysis.

Bradford reagent (Sigma) could be used to determine the final protein concentration in the extracts.

*EMSA:* For Electrophoretic mobility shift assays an oligonucleotide representing NF-κB binding sequence such as (5'-AGC TCA GAG GGG GAC TTT CCG AGA G-3') are synthesized. Hundred pico mol sense and antisense oligo are annealed and labeled with γ- ³²P-DATP using T4 polynucleotide kinase according to manufacture's instructions (Promega, Madison, WI). Cytosolic extract or nuclear extract (5-7.5 µg) from cells treated with regulatory peptide or from untreated cells is incubated for 30 minutes with 75000 cpm probe in binding reaction mixture (20 microl) containing 0.5 µg poly dI-dC (Amersham Pharmacia Biotech) and binding buffer BSB (25 mM MgCl₂, 5 mM CaCl₂, 5mM DTT and 20% Ficoll) at room temperature. Or cytosolic and nuclear extract from untreated cells or from cells treated with stimuli could also be incubated with probe in binding reaction mixture and binding buffer. The DNA-protein complex is resolved from free oligonucleotide by electrophoresis in a 4-6% polyacrylamide gel (150 V, 2-4 hours). The gel is then dried and exposed to x-ray film. Peptides can be biotinylated and incubated with cells. Cells are then washed with phosphate-buffered saline, harvested in the absence or presence of certain stimulus (LPS, PHA, TPA, anti-CD3, VEGF, TSST-1, VIP or know drugs etc.). After culturing cells are lysed and cells lysates (whole lysate, cytosolic fraction or nuclear fraction) containing 200 micro gram of protein are incubated with 50 miroliter Neutr-Avidin-plus beads for 1 h at 4°C with constant shaking. Beads are washed five times with lysis buffer by centrifugation at 6000 rpm for 1 min. Proteins are eluted by incubating the beads in 0.05 N NaoH for 1 min at room temperature to hydrolyze the protein-peptide linkage and analyzed by SDS-polyacrylamide gel electrophoresis followed by immunoprecipitated with agarose-conjugated anti-NF-kB subunits antibody or immunoprecipitated with antibody against to be studied target. After hydrolyzing the protein-peptide linkage, the sample could be analyzed on HPLS and mass-spectrometry. Purified NF-kB subunits or cell lysate interaction with biotinylated regulatory peptide can be analysed on biosensor technology. Peptides can be labeled with FITC and incubated with cells in the absence or presence of different stimulus. After culturing, cells can be analysed with fluorescent microscopy, confocal microscopy, flow cytometry (cell membrane staining and/or intracellular staining) or cells lysates are made and analysed on HPLC and mass-spectrometry. NF-kB transfected (reporter gene assay) cells and gene array technology can be used to determine the regulatory effects of peptides.

*HPLC and mass-spectrometry analysis:* Purified NF-kB subunit or cytosolic/nuclear extract is incubated in the absence or presence of (regulatory) peptide is diluted (2:1) with 8 N guanidinium chloride and 0.1% trifluoracetic acid, injected into a reverse-phase HPLC column (Vydac C18) equilibrated with solvent A (0.1% trifluroacetic acid), and eluted with a gradient of 0 to 100% eluant B (90% acetonitrile in solvent A). Factions containing NF-kB subunit are pooled and concentrated. Fractions are then dissolved in appropriate volume and could be analysed on mass-spectrometry.

Acute phase proteins (APP) or acute phase reactants (APR) is the generic name given to a group of approximately 30 different biochemically and functionally unrelated proteins. The levels of acute phase proteins in the serum are either increased (positive acute phase reactants) or reduced (negative acute phase reactants) approximately 90 minutes after the onset of an inflammatory reaction. The more important acute phase proteins are usually lycoproteins. Exceptions are C-reactive protein (CRP) and serum amyloid A protein (SAA).

Acute phase proteins of inflammation and their function include:
alpha-1 acid glycoprotein (interaction with collagen, promotion of fibroblast growth,binding of certain steroids), alpha-1 antichymotrypsinogen (protease inhibitor), alpha-1 antitrypsin (protease inhibitor, resolution of emphysema), alpha-2 antiplasmin (modulation of coagulation cascade), alpha-2-Macroglobulin (inhibitor of several serum proteases and other functions), antithrombin-3 (modulation of coagulation cascade), C1 (inhibitor negative control of complement cascade), C2, C4, C4 binding protein, C5 and C9 as complement component, C-reactive protein (binding to membrane phosphorylcholine, complement activation and opsonization, interaction with T-cells and B-cells), Ceruloplasmin (copper transport protein, reactive oxygen scavenger), Factor VIII (clotting formation of fibrin matrix for repair), Factor-B complement component), Ferritin (iron transport protein), Fibrinogen (clotting formation of fibrin matrix for repair), Fibronectin (fibrin clot formation), Haptoglobin (hemoglobin scavenger), Heme oxygenase (heme degradation), Hemopexin (heme binding and transport protein), Heparin cofactor-2 (proteinase inhibitor), Kallikreins (vascular permeability and dilatation), LPS binding protein (macrophage cell activation), Manganese superoxide dismutase (copper zinc binding protein, formation of reactive oxygen species), Mannose-binding protein (serum lectin), Plasminogen (proteolytic activation of complement, clotting, fibrinolysis), Plasminogen activator inhibitor-1 (protease inhibitor), Prothrombin (clotting formation of fibrin matrix for repair), Serum amyloid A (cholesterol and HDL scavenger), Serum amyloid-P (formation of IgG immune complexes), von Willebrand factor (coagulation protein), IL1ra (IL1 receptor antagonist).

IL1ra (IL1 receptor antagonist) has been shown recently to be regulated by various pro-inflammatory cytokines in the same way as other acute phase proteins.

Acute phase proteins are synthesized predominantly in the liver with each hepatocyte possessing the capacity to produce the entire spectrum of these proteins. Following stimulation of single hepatocytes within individual lobules one observes a stimulation of further hepatocytes and this process continues until almost all hepatocytes produce these proteins and release them into the circulation. The various acute phase proteins differ markedly in the rise or decline of their plasma levels and also in their final concentrations. Nevertheless, acute phase responses generate a characteristic serum protein profile. Levels of elevated expression can differ widely from species to species and some proteins that function as an acute phase protein in one species may not be an acute phase protein in another species.

Acute phase proteins regulate immune responses, function as mediators and inhibitors of inflammation, act as transport proteins for products generated during the inflammatory process (the heme-binding protein hemopexin, and Haptoglobin), and/or play an active role in tissue repair and remodeling (Wound healing). Van Molle et al suggest that at least some acute phase proteins might constitute an inducible system of factors protecting against cell death by apoptosis. They observe that alpha1-acid glycoprotein and alpha1-antitrypsin activate the major executioners of apoptosis, caspase-3 and caspase-7.

Some of the acute phase proteins behave like cytokines. C-reactive protein, for example, activates macrophages (MAF, macrophage activating factor) while some other acute phase proteins influence the chemotactic behavior of cells (MIF, migration inhibition factor). Some acute phase proteins possess antiproteolytic activity and presumably block the migration of cells into the lumen of blood vessels thus helping to prevent the establishment of a generalized systemic Inflammation. A failure to control these processes, i. e., an uncontrolled acute phase reaction, eventually has severe pathological consequences such as Systemic inflammatory response syndrome.

The elevated serum concentrations of certain acute phase proteins are of diagnostic relevance and also of prognostic value. Their measurement, for example, allows inflammatory processes to be distinguished from functional disturbances with similar or identical clinical pictures. Under normal circumstances an acute phase response is not observed with functional disturbances that are not the result of an inflammatory process, thereby allowing the differentiation between failure of function and organic disease.

Some acute phase reactions are observed also in chronic disorders such as rheumatoid arthritis and chronic infections. There are many diseases in which the rise in the synthesis of acute phase proteins parallels the degree and progression of the inflammatory processes.

The co-ordinated expression of many acute phase proteins as a direct consequence of the activities of several cytokines can be explained, at least in part, by the fact that the regulatory sequences of the genes encoding these acute phase proteins contain so-called cytokine response elements (for example IL6RE as an IL6-specific element). These elements are recognized specifically by transcription factors that mediate the activity of these genes in a cell- and/or tissue-specific manner.

The major inducers of acute phase proteins are IL1, IL6, and TNF. The two mediators IL1 and IL6 have been used to classify acute phase proteins into two subgroups. Type-1 acute phase proteins are those that require the synergistic action of IL6 and IL1 for maximum synthesis. Examples of Type-1 proteins are C-reactive protein, serum amyloid A and alpha-1 acid glycoprotein. Type-2 acute phase proteins are those that require IL6 only for maximal induction. Examples of Type-2 proteins are fibrinogen chains, haptoglobin, and alpha-2-Macroglobulin. Expression of genes encoding Type-2 acute phase proteins is suppressed rather than being enhanced frequently by IL1 (Ramadori et al; Fey et al). Additive, synergistic, co-operative, and antagonistic effects between cytokines and other mediator substances influencing the expression of acute phase proteins do occur and have been observed in almost all combinations. Many cytokines also show differential effects, inducing the synthesis of one or two acute phase proteins but not others. For example, Activin A induces a subset of acute phase proteins in HepG2 cells. Bacterial lipopolysaccharides and several cytokines (mainly IL1, IL6 and TNF but also LIF, CNTF, oncostatin M, IL11, and cardiotrophin-1) are involved in the induction of SAA synthesis and some of these cytokines act synergistically.

IL1 and also IFN-gamma reduce some of the effects of IL6. Some of the effects of IL2 and IL6 are antagonized by TGF-beta. The combined action of two or even more cytokines may produce effects that no factor on its own would be able to achieve. In cultured HepG2 hepatoma cells IL1, IL6, TNF-alpha and TGF-beta induce the synthesis of antichymotrypsin and at the same time repress the synthesis of albumin and AFP (alpha-Fetoprotein). The synthesis of fibrinogen is induced by IL6 and this effect is, in turn, suppressed by IL1-alpha, TNF-alpha or TGF-beta-1. The increased synthesis of Haptoglobin mediated by IL6 is suppressed by TNF-alpha. Insulin inhibits the synthesis of some negative acute phase proteins (prealbumin, Transferrin, and fibrinogen, in HepG2 hepatoma cells. For further information see also: Acute phase reaction, Inflammation, and Systemic inflammatory response syndrome.

The fact that different patterns of cytokines are involved in systemic and localized tissue damage is supported by obervations with knock-out mice for IL1 and IL6. Inflammatory acute phase response after tissue damage or infection is severely compromised in IL6 knock-out mice, but only moderately affected after challenge with bacterial lipopolysaccharides. Also, in the absence of IL6, the induction of acute phase proteins is dramatically reduced in response to turpentine injections but those parameters are altered to the same extent both in wild-type and IL6-deficient mice following injection of bacterial lipopolysaccharides. These mice, however, produce three times more TNF-alpha than wild-type controls. Also, a normal acute phase reaction was observed to both turpentine and bacterial lipopolysaccharides in TNF-beta knock-out mice. There is, however, a striking absence of elevated major acute phase proteins, SAP and SAA, in mice deficient in TNF-beta and IL6.

Regulation of acute phase reactions and Synthesis of acute phase proteins.

Inflammatory cytokines such as IL6, IL1, TNF, and others such as TGF, IFN, and LIF are produced by inflammatory cells. They induce local and systemic reactions. Among other things these mediators are involved in cell activation of leukocytes, fibroblasts, endothelial cells, and smooth muscle cells, inducing the synthesis of further cytokines. These mediators also have direct actions in hepatocytes of the liver. Activities are enhanced indirectly by activation of the pituitary/adrenal gland axis which involves synthesis of ACTH and subsequent production of cortisol. Cortisol can enhance expression of IL6 receptors in liver cells and thus promotes IL6 mediated synthesis of acute phase proteins.

Negative regulatory loops can involve inhibition of synthesis of IL6, IL1, and TNF by cortisol and inhibition of the synthesis of IL1 and TNF in monocytes by IL6. Of all mediators participating in the induction and regulation of acute phase protein synthesis IL6 appears to induce the broadest spectrum of acute phase proteins whereas IL1 and TNF only induce the synthesis of subsets of these proteins.

Almost all cytokines are pleiotropic effectors showing multiple biological activities. In addition, multiple cytokines often have overlapping activities and a single cell frequently interacts with multiple cytokines with seemingly identical responses (cross-talk). One of the consequences of this functional overlap is the observation that one factor may frequently functionally replace another factor altogether or at least partially compensate for the lack of another factor.

Many cytokines show stimulating or inhibitory activities and may synergise or antagonize also the actions of other factors. A single cytokine may elicit reactions also under certain circumstances which are the reverse of those shown under other circumstances. The type, the duration, and also the extent of cellular activities induced by a particular cytokine can be influenced considerably by the microenvironment of a cell, depending, for example, on the growth state of the cells (sparse or confluent), the type of neighboring cells, cytokine concentrations, the combination of other cytokines present at the same time, and even on the temporal sequence of several cytokines acting on the same cell. Under such circumstances combinatorial effects thus allow a single cytokine to transmit diverse signals to different subsets of cells.

The fact that every cell type may have different responses to the same growth factor can be explained, at least in part, by different spectrums of genes expressed in these cells and the availability and levels of various transcription factors that drive Gene expression. The responses elicited by cytokines are therefore contextual and the "informational content", i. e. the intrinsic activities of a given cytokine may vary with conditions. Although a variety of cytokines are known to share at least some biological effects the observations that single cells usually show different patterns of gene expression in response to different cytokines can be taken as evidence for the existence of cytokine receptor-specific signal transduction pathways. Shared and different transcriptional activators that transduce a signal from a cytokine receptor to a transcription regulatory element of DNA are involved in these processes (for example, STAT proteins, Janus kinases, IRS).

It has been observed, for example, that bFGF is a strong mitogen for fibroblasts at low concentrations and a chemoattractant at high concentrations. bFGF has been shown also to be a biphasic regulator of human hepatoblastoma-derived HepG2 cells, depending upon concentration. The interferon IFN-gamma can stimulate the proliferation of B-cells prestimulated with Anti-IgM, and inhibits the activities of the same cells induced by IL4. On the other hand, IL4 activates B-cells and promotes their proliferation while inhibiting the effects induced by IL2 in the same cells. The activity of at least two cytokines (IL1-alpha and IL1-beta) is regulated by an endogenous receptor antagonist, the IL1 receptor antagonist (IL1ra). Several cytokines, including TNF, IFN-gamma, IL2 and IL4, are inhibited by soluble receptors. Several cytokines, including IL10 and TGF-beta, act to inhibit other cytokines.

### Further references:

WO99/59617, WO01/72831, WO97/49721, WO01/10907, WO01/11048.

## Claims

1. A method for treating a subject suspected to suffer from a disease comprising an inflammatory condition and/or a counter-inflammatory condition comprising subjecting said subject to a diagnostic process aimed at determining inflammatory disease stage of said subject further comprising providing said subject with a gene-regulatory peptide or functional analogue thereof depending on the outcome of said determination of disease stage.

2. A method according to claim 1 wherein said diagnostic process includes determining the level of a pro-inflammatory cytokine.

3. A method according to claim 2 wherein said pro-inflammatory cytokine is selected from the group of tumor necrosis factor-alpha, interferon-gamma, interleukin-1-beta and interleukin-6.

4. A method according to any one of claims 1 to 3 wherein said diagnostic process includes determining the level of a counter-inflammatory cytokine.

5. A method according to claim 4 wherein said counter-inflammatory cytokine is selected from the group of interleukin-4 and interleukin-10.

6. A method according to any one of claims 1 to 5 wherein said diagnostic process includes determining HLA-DR expression on circulating monocytes of said subject.

7. A method according to any one of claims 1 to 6 wherein said diagnostic process includes determining arachidonic acid metabolite levels in said subject.

8. A method according to any one of claims 1 to 6 wherein said diagnostic process includes plasma prostaglandin levels in said subject.

9. A method according to claim 8 comprising determining a ratio between prostaglandins 1 and 2 (PGE1 and PGE2).

10. A method according to any one of claims 1 to 9 comprising providing said subject with a gene-regulatory peptide or functional analogue thereof based on the determination of disease stage as an essentially inflammatory condition.

11. A method according to claim 10 wherein said gene-regulatory peptide down-regulates translocation and/or activity of pro-inflammatory cytokine gene expression mediated by a gene transcription factor.

12. A method according to claim 11 wherein said gene transcription factor comprises an NF-kappaB/Rel protein.

13. A method according to claim 12 wherein translocation and/or activity of said NF-kappaB/Rel protein is inhibited.

14. A method according to any one of claims 10 to 14 wherein said peptide is selected from the group of peptides having NFkappaB down-regulating activity in LPS stimulated RAW264.7 cells.

15. A method according to any one of claims 10 to 14 wherein said peptide is selected from the group of peptides having NFkappaB down-regulating activity in LPS unstimulated RAW264.7 cells.

16. A method according to any one of claims 1 to 9 comprising providing said subject with a gene-regulatory peptide or functional analogue thereof based on the determination of disease stage as an essentially counter-inflammatory condition.

17. A method according to claim 16 wherein said gene-regulatory peptide up-regulates translocation and/or activity of pro-inflammatory cytokine gene expression mediated by a gene transcription factor.

18. A method according to claim 17 wherein said gene transcription factor comprises an NF-kappaB/Rel protein.

19. A method according to claim 18 wherein translocation and/or activity of said NF-kappaB/Rel protein is activated.

20. A method according to any one of claims 15 to 19 wherein said peptide is selected from the group of peptides having NFkappaB up-regulating activity in LPS un-stimulated RAW264.7 cells.

21. A method according to any one of claims 15 to 19 wherein said peptide is selected from the group of peptides having NFkappaB up-regulating activity in LPS stimulated RAW264.7 cells.

22. Use of a gene-regulatory peptide or functional analogue thereof for the production of a pharmaceutical composition for the treatment of a disease comprising an inflammatory condition and/or a counter-inflammatory condition wherein a subject suffering from said disease is subjected to a diagnostic process aimed at determining inflammatory disease stage of said subject and where treatment is selected depending on the outcome of said determination of disease stage.

23. Use according to claim 22 wherein said diagnostic process includes determining the level of a pro-inflammatory cytokine.

24. Use according to claim 23 wherein said pro-inflammatory cytokine is selected from the group of tumor necrosis factor-alpha, interferon-gamma, interleukin-1-beta and interleukin-6.

25. Use according to any one of claims 22 to 24 wherein said diagnostic process includes determining the level of a counter-inflammatory cytokine.

26. Use according to claim 25 wherein said counter-inflammatory cytokine is selected from the group of interleukin-4 and interleukin-10.

27. Use according to any one of claims 22 to 26 wherein said diagnostic process includes determining HLA-DR expression on circulating monocytes of said subject.

28. Use according to any one of claims 22 to 27 wherein said diagnostic process includes determining arachidonic acid metabolite levels in said subject.

29. Use according to any one of claims 22 to 28 wherein said diagnostic process includes plasma prostaglandin levels in said subject

30. Use according to any one of claims 22 to 29 comprising providing said subject with a gene-regulatory peptide or functional analogue thereof based on the determination of disease stage as an essentially inflammatory condition.

31. Use according to claim 30 wherein said gene-regulatory peptide down-regulates translocation and/or activity of pro-inflammatory cytokine gene expression mediated by a gene transcription factor.

32. Use according to any one of claims 30 to 32 wherein said peptide or analogue is selected from the group of peptides or analogues having NFkappaB down-regulating activity in LPS stimulated RAW264.7 cells.

33. Use according to any one of claims 22 to 29 comprising providing said subject with a gene-regulatory peptide or functional analogue thereof based on the determination of disease stage as an essentially counter-inflammatory condition.

34. A method according to claim 33 wherein said gene-regulatory peptide up-regulates translocation and/or activity of pro-inflammatory cytokine gene expression mediated by a gene transcription factor.

35. Use according to any one of claims 33 to 34 wherein said peptide is selected from the group of peptides having NFkappaB up-regulating activity in LPS un-stimulated RAW264.7 cells.
